# EUROPEAN PATENT APPLICATION

(11) **EP 1 393 678 A1**
(43) Date of publication of application: **03.03.2004**
(21) Application number: 02736013.0
(22) Date of filing: 06.06.2002
(51) Int. Cl.: A61B 5/15, B23Q 3/18

(54) **EJECTING DEVICE**

(30) Priority: 07.06.2001 JP 2001172657
(71) Applicant: Arkray, Inc., Kyoto-shi, Kyoto 601-8045 (JP)
(72) Inventor: KASAI, Tokuo, c/o ARKRAY, INC., Kyoto-shi, Kyoto 601-8045 (JP); SATO, Yoshiharu, c/o ARKRAY, INC., Kyoto-shi, Kyoto 601-8045 (JP)
(74) Representative: Piésold, Alexander J.
(86) International application number: PCT/JP2002/005579
(87) International publication number: WO 2002/100273

(57) **Abstract**

A protrusible tool 14 is attached to an end portion of a movable member 1, and shoulder parts 11, 12, and 13 that have different heights are provided at the end portion of the movable member. The movable member 1 is set in the moving device 3, and a detachable cap 2 is put on the movable member 1. A through hole 22 that allows the protrusible tool 14 to pass threrethrough is formed at an end portion of the cap 2, and projections 21 that project toward center of the through hole 22 are formed. The movable member 1 is ejected and moved by the moving device 3, and the shoulder parts 11, 12, or 13 are brought into contact with the projections 21, whereby the length of a portion of the protrusible tool 14 protruded out of the cap 2 is regulated.

## Description

### TECHNICAL FIELD

The present invention relates to tool-protruding devices useful as tools for cutting and perforating, and medical instruments for puncture. It particularly relates to a lancet assembly that is used for blood sampling.

### BACKGROUND ART

Conventionally, many tools provided with members on their tips for cutting or perforating an object to be processed have been known, and have been used in various fields. In processing operations using such tools, it is necessary in many cases to adjust the extent to which the object is processed. For instance, in a cutting operation, a cutting amount needs to be adjusted in some cases, and in a perforating operation, a depth of a hole needs to be adjusted in some cases.

Here, the following describes a tool, referring to a case where a perforating operation is carried out using a drill. Normally, a drill is composed of a shank and a body having a cutting edge. The drill is fixed by inserting the shank into a chuck, and covering the chuck with a cover so that the chuck is fastened by the cover. Here, by adjusting the length of the drill protruded from the chuck, the depth of a hole to be formed can be adjusted.

However, for adjusting the length of the drill, a measuring instrument and the like are needed, and the user is required to perform complex tasks. Further, for instance, in the case where the object to be processed is a thin plate and the hole to be formed must not go through the plate, the adjustment of the length of the drill is significantly subtle and difficult.

Further, a medical instrument is described below, with reference to a case where puncture is carried out using a lancet. A diabetic has to measure a blood glucose level for him/herself, and for this purpose, he/she normally samples blood for him/herself using a disposable lancet. A lancet generally has a structure such that a needle or a cutting edge is attached at an end of a cylindrical body. The lancet normally is used in a state of being set in a lancet device (normally, a combination of a lancet device and a lancet set in the lancet device is called a "lancet assembly").

The lancet device is composed of a moving device (hereinafter referred to as "lancet device body") that ejects the lancet and moves the same in a lengthwise direction, and a cap. The cap is put on the lancet and is fixed to the lancet device body after the lancet is set in the device.

At an end of the cap, there is a through hole, which is a small hole that allows a needle or a cutting edge of the lancet to pass therethrough. Therefore, when the lancet is ejected, an end of the cylindrical body collides against the cap. This allows only a portion of a predetermined length of the needle or the cutting edge to protrude out of the cap, and prick the skin to a predetermined depth. The device is configured so that the needle or the cutting edge thus protruding automatically returns into the device.

Here, the depth through which the lancet penetrates when pricking (hereinafter referred to as penetration depth), that is, the length of the protruded portion of the needle or the cutting edge of the lancet, is important, since if it is too shallow, only an amount of blood insufficient for measurement comes out, whereas if it is too deep, it causes more bleeding than necessary and inflicts pain on a patient.

In a normal lancet device, the penetration depth in the skin (protrusion length of the needle or the cutting edge) can be adjusted at one to four levels. For instance, a scheme is available in which several types of caps varying in length from a long one to a short one are prepared, and one is selected for use according to the penetration depth (cap-changing scheme). Apart from this, another scheme is available in which the cap has a duplex structure and the distance of the inside of the cap is adjusted by moving an external part of the cap forward or backward by turning the cap leftward or rightward (as disclosed by, for instance, WO97/04707, and H10(1998)-508527A).

However, in the case of the cap-changing scheme, the work of the user who changes the caps increases, and the number of components increases, thereby leading to a problem in the manufacturing efficiency. Further, in the case of the scheme using the cap with a duplex structure, the complexity of the cap structure also makes the manufacturing process complex.

The present invention was made in light of the foregoing problems, and it is an object of the present invention to provide a tool-protruding device that has a simple structure and that is configured so that a protrusion length of an instrument can be adjusted easily by a user.

### DISCLOSURE OF THE INVENTION

To achieve the foregoing object, a tool-protruding device includes at least a movable member, a moving device for moving the movable member, and a cap attached to the moving device and covering the movable member. In the tool-protruding device, the movable member has an end that has a shoulder part having a varied height and that is configured so that a protrusible tool is attachable thereto. The cap has a through hole that allows the protrusible tool to pass threrethrough, and one or a plurality of projections that project toward center of the through hole, so that the shoulder part of the movable member is brought into contact with the projections, so as to regulate a length of a portion of the protrusible tool protruded out of the cap.

In the tool-protruding device, by selecting a portion of the shoulder part of the movable member to be brought into contact with the projections that project toward the center of the through hole of the cap, the protrusion length of the protrusible tool can be adjusted easily without performing complex works such as changing caps. Further, since the tool-protruding device has a simple structure, the shoulder part having a varied height, the projections of the cap, etc. can be formed easily by preparing a die or the like suitable for forming the same. It should be noted that the cap may be detachable with respect to the moving device.

In the tool-protruding device of the present invention, the height of the shoulder part of the movable member may be varied continuously or stepwise, but it is preferable that the height of the shoulder part is varied stepwise, for the reasons, for instance, that the protrusion length can be adjusted more surely. In this case, the height of the shoulder part is varied stepwise at, for instance, two to twelve levels, preferably two to five levels, more preferably two to three levels.

Further, the manner of the stepwise variation of the height of the shoulder part may be continuous or discontinuous. The continuous stepwise variation indicates the case where shoulder part portions having different height levels are arranged orderly from high to low. Further, the discontinuous stepwise variation indicates the case where shoulder part portions having various levels of height are arranged at random, irrespective of the levels of the height.

In the case where the height of the shoulder part is varied stepwise, the following configuration is preferable. The movable member has a plurality of projected portions (normally referred to as "ribs") that linearly extend in a direction in which the movable member is moved and that correspond to levels of the height of the shoulder part that is varied stepwise, respectively. The moving device has a groove into which one of the projected portions is to be fitted. The shoulder part, at a position where it has a predetermined level of the height that corresponds to one of the projected portions fitted in the groove, is brought into contact with the projections formed in the cap, by causing the moving device to move the movable member in a state in which the projected portion is fitted in the groove.

With this configuration, a portion of the shoulder part that has the intended height can be brought into contact with the projections more surely. It should be noted that the projected portion and the groove may be provided inversely. In other words, grooves may be formed on a circumferential surface of the movable member in place of the projected portions, and a projected portion may be formed on the moving device in place of the groove, so that the projected portion of the moving device is fitted in the groove of the movable member.

In the tool-protruding device of the present invention, examples of the protrusible tool attached to the end of the movable member include, for instance, a chisel, a sharpening stone, a plane, a scraper, a drill, a saw, a wrench, a cutter, a file, and a reamer.

In the case where the tool-protruding device of the present invention is used as a lancet assembly, examples of the protrusible tool include a needle or a cutting edge for a lancet. In this case, the movable member is a cylindrical body composing the lancet, the moving device is a device body composing a lancet-ejecting device, and the cap is a cap composing the lancet-ejecting device.

Further, in the case where the tool-protruding device of the present invention is used as a lancet assembly and the protrusible tool is a needle or a cutting edge for a lancet, a length of a portion of the protrusible tool protruded out of the cap is, for instance, in a range of 0.0 mm to 4.0 mm, preferably in a range of 0.2 mm to 3.3 mm, more preferably in a range of 0.2 mm to 1.0 mm.

Still further, in this case, the tool-protruding device may be configured so that the protrusible tool is not protruded at all out of the cap (in other words, the protrusion length is 0 mm). In the case of this configuration, by pressing a finger pulp against the end of the cap relatively strongly, the finger pulp bulges to the inside of the through hole and projects thereto. Therefore, by ejecting the moving member in this state, the needle or the cutting edge is caused to prick the finger, even if the protrusion length is 0 mm. In the case where it is not desired that the needle or the cutting edge penetrates deeply, the foregoing method may be applied in practice.

### BRIEF DESCRIPTION OF DRAWINGS

FIG. 1 is a perspective view illustrating a tool-protruding device according to Embodiment 1 of the present invention.
FIG. 2 is a perspective view illustrating a movable member composing the tool-protruding device according to Embodiment 1 of the present invention.
FIG. 3 is a perspective view illustrating a cap composing the tool-protruding device according to Embodiment 1 of the present invention.
FIG. 4 is a perspective view illustrating a state in which shoulder parts of the movable member are brought into contact with the cap in Embodiment 1.
FIG. 5 is a perspective view illustrating a state in which shoulder parts of the movable member are brought into contact with the cap in Embodiment 1.
FIG. 6 is a perspective view illustrating a state in which shoulder parts of the movable member are brought into contact with the cap in Embodiment 1.
FIG. 7 is a view illustrating an example in which projections of the cap have different heights.
FIG. 8 is a perspective view illustrating an example of a tool-protruding device according to Embodiment 2 of the present invention.
FIG. 9 is a perspective view illustrating another example of the tool-protruding device according to Embodiment 2 of the present invention.
FIG. 10 is a perspective view illustrating still another example of the tool-protruding device according to Embodiment 2 of the present invention.
FIG. 11 is a perspective view illustrating still another example of the tool-protruding device according to Embodiment 2 of the present invention.
FIG. 12 is a perspective view illustrating still another example of the tool-protruding device according to Embodiment 2 of the present invention.

### DESCRIPTION OF THE INVENTION

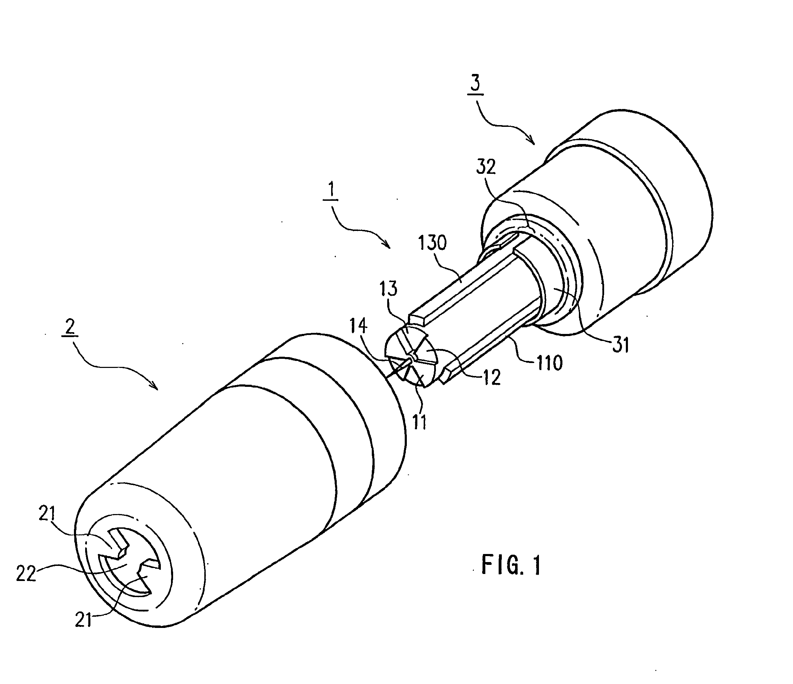

The following will describe a tool-protruding device of the present invention, while referring to FIGs. 1 to 12. In these drawings, the same members are designated with the same reference numerals.

### Embodiment 1

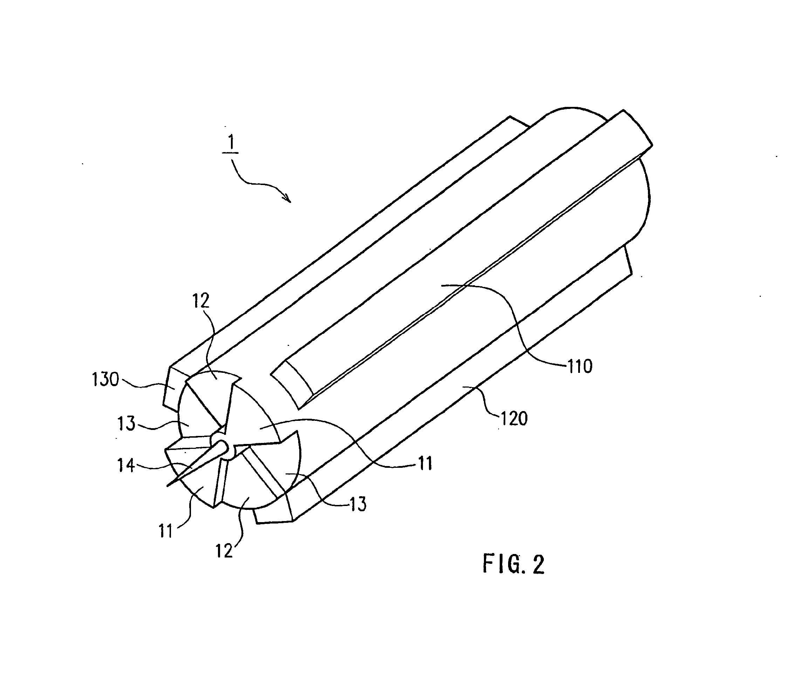

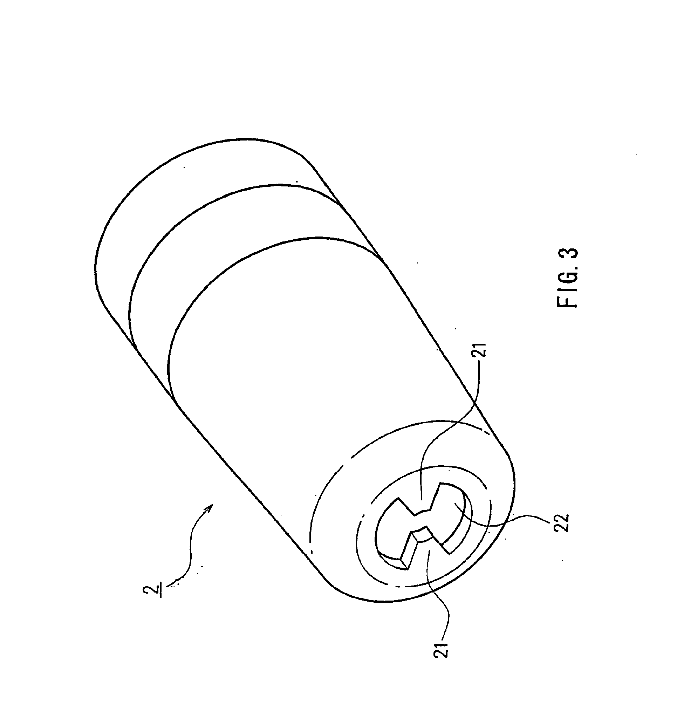

First of all, a tool-protruding device according to Embodiment 1 of the present invention is described. FIG. 1 is a perspective view illustrating the tool-protruding device according to Embodiment 1 of the present invention. FIG. 2 is a perspective view illustrating a movable member composing the tool-protruding device according to Embodiment 1 of the present invention. FIG. 3 is a perspective view illustrating a cap composing the tool-protruding device according to Embodiment 1 of the present invention.

As shown in FIG. 1, the tool-protruding device according to Embodiment 1 includes at least a movable member 1, a moving device 3 for moving the movable member 1 in a lengthwise direction, and a cap 2 covering the movable member 1. In Embodiment 1, the movable member is formed in a cylindrical shape, and an end of the movable member 1 on one side (end portion) is configured so that a protrusible tool 14 is attachable thereto. In the example of FIG. 1, the protrusible tool 14 is attached to the foregoing end. The other end of the movable member 1 is set in the moving device 3. The cap 2 is attached to the moving device 3 so as to cover the movable member 1.

In the present embodiment 1, the tool-protruding device is a lancet assembly as a medical instrument. In the present embodiment 1, a lancet is composed of the movable member 1 and the protrusible tool 14. In other words, the movable member is a cylindrical body constituting the lancet, and the protrusible tool 14 is a needle for puncture (for lancet). Further, the moving device 3 and the cap 2 compose a lancet-ejecting device, and the moving device 3 is a device body having an ejecting function.

In the present embodiment 1, in the end portion of the movable member 1, a hole through which a needle as the protrusible tool 14 is inserted is provided so as to allow the protrusible tool 14 to be attachable. It should be noted that the description "the protrusible tool is attachable" herein does not exclusively indicate the case where the movable member 1 is configured so that the protrusible tool 14 manufactured separately from the movable member 1 can be attached thereto afterward, but the cases indicated by the description include the case where the movable member 1 is manufactured in a state in which the protrusible tool 14 already is attached thereto. For instance, the description also indicates a case in which, in the case where the movable member 1 is made of a resin or the like by molding, the protrusible tool 14 is placed in a die and the movable member 1 is molded integrally with the protrusible tool 14 so that the protrusible tool 14 is fixed to the movable member 11.

The moving device 3 includes an elastic body (not shown) inside, and using the elastic body, the moving device 3 ejects the movable member 1 toward the end portion and moves the movable member 1 in the lengthwise direction thereof. Further, the moving device includes another elastic body that returns the ejected movable member to an original position. In the present embodiment 1, coil springs are used as these elastic bodies.

It should be noted that in the present invention, the moving device 3 is not limited to this embodiment. For instance, the moving device 3 may be configured so that the user is allowed to arbitrarily return the movable member to the original position, or may be configured so that the application of a force by the user causes the movable member to return to the original position. Further, it may be configured so that the movable member ejected can be maintained at a position thereof after the ejection.

As shown in FIGs. 1 and 2, shoulder parts 11 to 13 are provided at the end portion of the movable member 1. In the present embodiment 1, the shoulder parts 11 to 13 compose one shoulder part that has a varied height. In the present embodiment 1, two shoulder parts 11 that are highest, two shoulder parts 12 that have an intermediate height, and two shoulder parts 13 that are lowest, are formed. The movable member 1 further includes, on its circumferential surface, three projected portions that extend linearly in a direction in which the movable member 1 is moved. One of the projected portions, denoted with 110, corresponds to the highest shoulder parts 11, another one of the projected portions, denoted with 120, corresponds to the shoulder parts 12 having the intermediate height, and the other one of the projected portions, denoted with 130, corresponds to the lowest shoulder parts 13.

Further, as shown in FIG. 1, a C-letter-shaped member 31 is arranged at a position in the moving device 3 where the movable member 1 is set, and one of the three projected portions 110, 120, and 130 of the movable member 1, which is a lancet, is fitted in a gap (groove) 32 of the C-letter-shaped member 31. Accordingly, the movable member 1 is moved using the gap 32 as a guide.

It should be noted that the shape of the movable member 1 in the present invention is not limited particularly, and it may be in any shape as long as the protrusible tool 14 is attachable thereto. More specifically, the movable member 1 preferably has a cylindrical shape, a prismatic shape (for instance, a triangular prism, a quadrangular prism, a pentangular prism, etc.), a pillar-like shape having a star-shaped cross section, a circular conic shape, or an angular conic shape. Alternatively, the movable member 1 may be in a plate-like shape, or in a bar-like shape. Further alternatively, the movable member 1 may be formed by using a plate-like material and a bar-like material in combination.

Further, the device may be configured so that the movable member 1 has a groove portion in place of the projected portion on the circumferential surface and the moving device 3 has a projected portion in place of the C-letter-shaped member 31 so that the projected portion is fitted in the groove of the movable member 1.

As shown in FIGs. 1 and 3, at an end portion of the cap 2, a through hole 22 is formed, which has a size such that the protrusible tool 14, which is the needle of the lancet, can pass therethrough. In the through hole 22, two projections 21 project out toward the center of the through hole 22. It should be noted that in the protruding device of the present invention, the number of the projections 21 is not limited particularly, and for instance, the number may be one, or alternatively, three or more.

In the present embodiment 1, when the movable member 1 is moved by the moving device 3, the two projections 21 come into contact with any ones of the shoulder parts 11 to 13 of the movable member 1. Thus, the protrusion length of a portion of the protrusible tool 14 protruding from the cap 2 is regulated. It should be noted that the protrusion length herein refers to a height of the protrusible tool 14 with respect to the end of the cap 2 as a reference.

The following describes the blood sampling employing the foregoing tool-protruding device that is the lancet assembly. First, the movable member 1 that is the lancet is set in the moving device 3 that is the device body in a state in which a predetermined projected portion (the projected portion 130 in FIG. 1) of the movable member 1 is fitted in the gap (groove) 32 of the C-letter-shaped member 31.

Upon the foregoing setting, a spring (not shown) housed in the moving device 3 becomes in a compressed or stretched state. Then, the cap 2 is put on the movable member 1. At this time, or when the movable member 1 is ejected after putting the cap 2, the positions of projections 21 are adjusted by turning the cap 2 or the like, so that shoulder parts (11, 12, or 13) having an intended height are brought into contact with the projections 21 of the cap 2. It should be noted that before ejecting the movable member 1, the shoulder parts (11, 12, or 13) are not in contact with the projections 21.

In this state, an end of the tool-protruding device (an end of the cap 2) is brought into contact with the body at a position where blood is to be sampled, and a button (not shown) of the moving device 3 is pressed so that the spring housed inside is released from the compressed or stretched state. By so doing, the movable member 1 is ejected, and moves in the lengthwise direction.

This causes the protrusible tool (needle for puncture) 14 attached to the movable member 1 to pass through the through hole 22 of the cap 2 and to prick the skin, but since the shoulder parts (11, 12 or 13) provided at the end of the movable member 1 are brought into contact with the projections 21, the protrusible tool does not advance further.

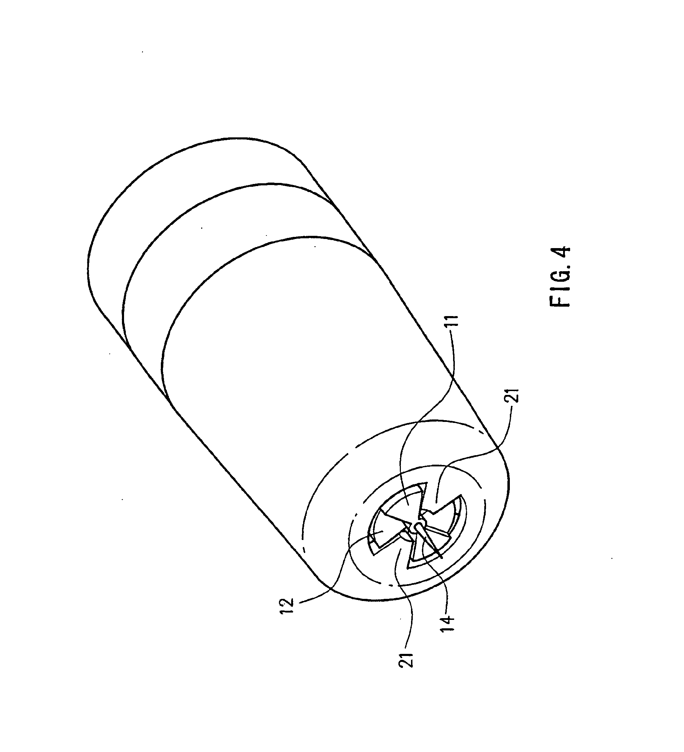

FIGs. 4 to 6 are perspective views illustrating states in which the shoulder parts of the movable member are in contact with the cap in the present embodiment 1.

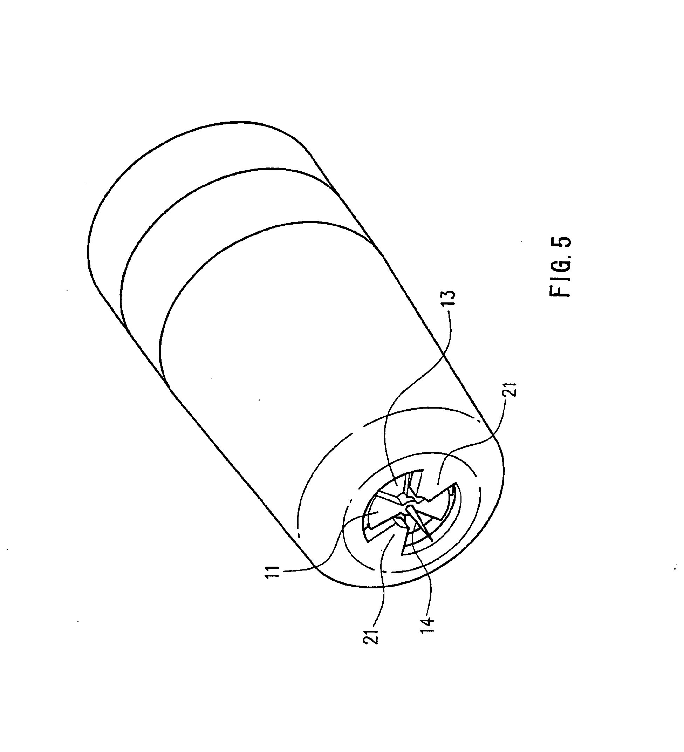

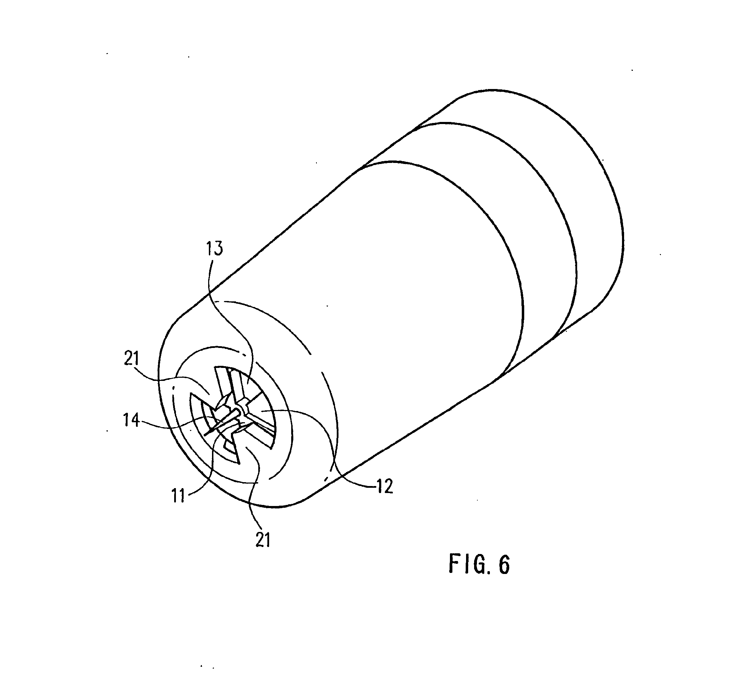

FIG. 4 illustrates a state in which the lowest shoulder parts 13 are in contact with the projections 21. In this case, a distance through which the protrusible tool 14 is protruded is longest, and therefore, the penetration depth is deepest. FIG. 5 illustrates a state in which the shoulder parts 12 having the intermediate height are in contact with the projections 21. In this case, the distance through which the protrusible tool 14 is protruded is intermediate, and therefore, the penetration depth is intermediate. FIG. 6 illustrates a state in which the highest shoulder parts 11 are in contact with the projections 21. In this case, the distance through which the protrusible tool 14 is protruded is shortest, and therefore, the penetration depth is shallowest.

Upon the ejection, since the projected portion (110, 120, or 130) on the circumferential surface of the movable member 1 is fitted in the gap (groove) 32 of the C-letter-shaped member of the device body, this structure functions as a guide, thereby ensuring that the intended shoulder parts come in contact with the projections 21. Then, after the protrusible tool 14 pricks the skin, the movable member 1 automatically is pulled back toward the moving device 3 due to an effect of another spring. Thereafter, the tool-protruding device is removed, blood is sampled from the bleeding portion, and a blood glucose level or the like is measured using a glucometer or the like.

Though the penetration depth is adjusted at three levels in the present embodiment 1, the present invention is not limited to this. Further, the other conditions such as the length of the needle as the protrusible tool 14 are determined appropriately according to the size and the use of the movable member 1 that is a lancet.

Further, though the protrusion length of the protrusible tool 14 is regulated in a manner such that the cap 2 itself is fixed, the movable member 1 is moved in the cap 2, and the shoulder parts (11, 12, or 13) collide against the projections 21 of the cap 2 in the present embodiment 1, the present invention is not limited to this. For instance, the tool-protruding device may be configured as follows. The shoulder parts (11, 12, or 13) of the movable member 1 are fixed in contact with the projections 21 of the cap 2 beforehand, whereby the protrusible tool 14 is protruded out of the cap 2 so as to have a predetermined protrusion length, and the movable member 1 together with the cap 2 is moved in this state so that the protrusible tool 14 pricks the skin.

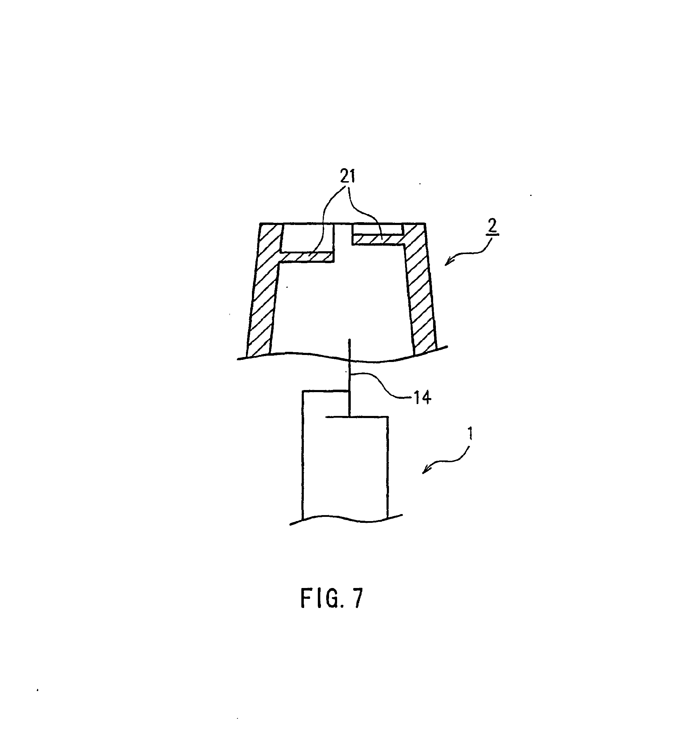

Further, though the two projections 21 of the cap 2 have equal heights in the present embodiment 1, the present invention is not limited to this. In the case where a plurality of projections are provided, they may vary in height. FIG. 7 is a view illustrating an example in which the projections of the cap have different heights. In the drawing, the same members as those of FIG. 1 are designated by the same reference numerals as those of FIG. 1. Further, the movable member 1 and the protrusible tool 14 are illustrated in a simplified manner.

As shown in FIG. 7, in the case where the plurality of projections 21 have different heights, selectable options of the penetration depth are increased according to the combinations of the heights of the shoulder parts of the movable member and the heights of the projections 21.

It should be noted that the use of the tool-protruding device according Embodiment 1, which is a lancet assembly, is not limited to the blood sampling by a diabetic him/herself, but the foregoing tool-protruding device is applicable to every type of blood sampling.

### Embodiment 2

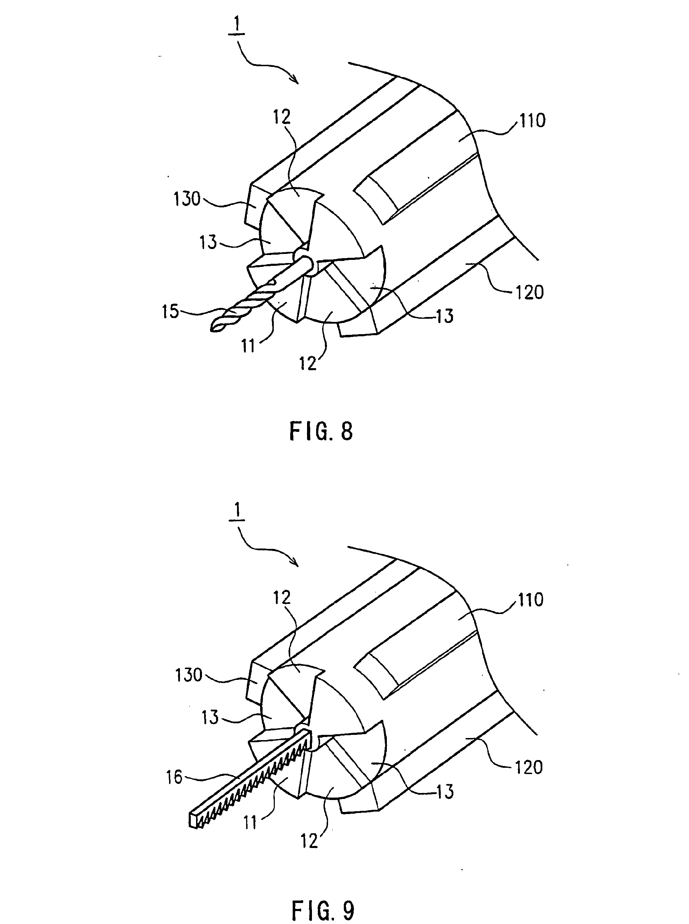

Next, the following will describe a tool-protruding device according to Embodiment 2 of the present invention. FIG. 8 is a perspective view illustrating an example of a tool-protruding device according to Embodiment 2 of the present invention. The foregoing drawing illustrates only an end of a movable member and a protrusible tool attached thereto.

As shown in FIG. 8, also in the present embodiment 2, like in Embodiment 1, shoulder parts 11 to 13 having different heights are provided at an end portion of the movable member 1, and projected portions 110 to 130 corresponding to these shoulder parts are provided on a circumferential surface of the movable member 1. Further, a C-letter-shaped member is arranged on a moving device (not shown) in which the movable member 1 shown in FIG. 8 is set, and the projected portion 110, 120, or 130 is fitted in a gap of the C-letter-shaped member. Still further, a cap used in the present embodiment 2 is identical to that shown in FIG. 3.

Therefore, in the present embodiment 2 also, when the movable member is moved by the moving device, projections of the cap are brought into contact with the shoulder parts 11, 12, or 13 of the movable member 1, whereby the protrusion length of a portion of the protrusible tool protruded out of the cap is regulated.

However, in the present embodiment 2, unlike in Embodiment 1, a drill 15 is attached as the protrusible member to the end portion of the movable member 1, in place of the needle.

It should be noted that though the drill 15 is fixed by fitting a shank (not shown) of the drill 15 into a hole provided at the end portion of the movable member 1 in FIG. 8, the present embodiment 2 is not limited to this. For instance, the device may be configured so that the movable member 1 has a chuck and the drill 15 is fixed by fastening the chuck. Further, as described above relating to Embodiment 1, the device may be configured so that the drill 15 is fixed in the movable member 1 by forming the movable member 1 integrally with the drill 15. For instance, in the case where the movable member 1 is formed with a resin by molding, the drill 15 may be placed in a die so that the movable member 1 is molded integrally with the drill 15.

Still further, in the present embodiment 2, the moving device (not shown) is identical to that of Embodiment 1 in the aspect that it moves the movable member 1 in a lengthwise direction thereof. However, it does not have an ejecting mechanism or a returning mechanism composed of an elastic body or the like, but instead, it has a feeding mechanism composed of a ball screw or the like. It should be noted that the feeding mechanism may be caused to operate manually by a user, or may be caused to operate by a power source such as a motor provided in the movable member. Further, unlike Embodiment 1, the foregoing moving device has a mechanism of holding the movable member 1 in a state in which the shoulder parts (11, 12, or 13) of the movable member 1 are in contact with the projections of the cap.

Next, a perforating process employing the tool-protruding device according to the present embodiment 2 is described below. First, the movable member 1 is set in the moving device in a state in which a predetermined projected portion (110, 120, or 130) of the movable member 1 is fitted in the gap of the C-letter-shaped member of the moving device.

Then, the cap is put on the moving device. At this time, or when a cutting edge of the drill 15 is protruded after setting the cap, the positions of projections of the cap are adjusted by turning the cap or the like so that shoulder parts (11, 12, or 13) having an intended height are brought into contact with the projections of the cap. By so doing, a distance through which the drill 15 is moved forward or backward is determined, and necessarily the length of a portion of the drill 15 protruded out of the cap is determined. It should be noted that in the present embodiment 2, unlike Embodiment 1, the shoulder parts may be in contact with the projections already before using the device.

Next, the user holds the tool-protruding device with the drill 15 being protruded out of the cap, brings the drill 15 in contact with a plate material or the like to be processed, and revolves the drill 15 together with the tool-protruding device, through which a perforating operation is performed. Here, since the length of a portion of the drill that protrudes out of the cap is predetermined, the user can make a hole with a desired depth only by revolving the drill 15 until the cap becomes in contact with the processing object.

It should be noted that though the whole tool-protruding device is held and the revolving is carried out manually in the present embodiment 2 as described above, alternatively the moving device 3 may be equipped with a mechanism that causes the movable member 1 to revolve together with the cap 2 so as to impart a revolving force to the drill 15. Further, to facilitate the user's manual revolving work, the tool-protruding device may be configured to have a ratchet structure.

Still further, in the present embodiment 2, another tool may be attached instead of the drill 15. FIGs. 9 to 12 are perspective views illustrating other examples of the tool-protruding device according to Embodiment 2 of the present invention. It should be noted that in FIGs. 9 to 12 also, only the end portion of the movable member and the protrusible tool attached thereto are shown.

In the example of FIG. 9, a saw 16 as the protrusible tool is attached to the movable member 1. The saw 16 shown in FIG. 9 is a kraft saw that is used for processing or expanding a hole already provided, or strictly adjusting the depth of a hole, and that also is called a fret saw. However, a saw other than these may be attached.

The processing using the tool-protruding device shown in FIG. 9 is carried out in the following manner, for instance. First, as in the example of FIG. 8, the movable member 1 is set in the moving device, the cap is put on the moving device, and the positions of the projections are adjusted by turning the cap so that the length of a portion of the saw 16 protruded out of the cap is determined.

Next, the user holds the tool-protruding device having the saw 16 protruded out of the cap, inserts the saw 16 into a hole to be processed, and carries out the work. Here also, as in the example of FIG. 8, the length of a portion of the saw 16 protruded out of the cap is predetermined, and the cap is brought into contact with surroundings of the hole to be processed, thereby functioning as a stopper. Therefore, the user is required to pay attention only to the expansion of the hole in diameter, without concern for the hole in the depth direction.

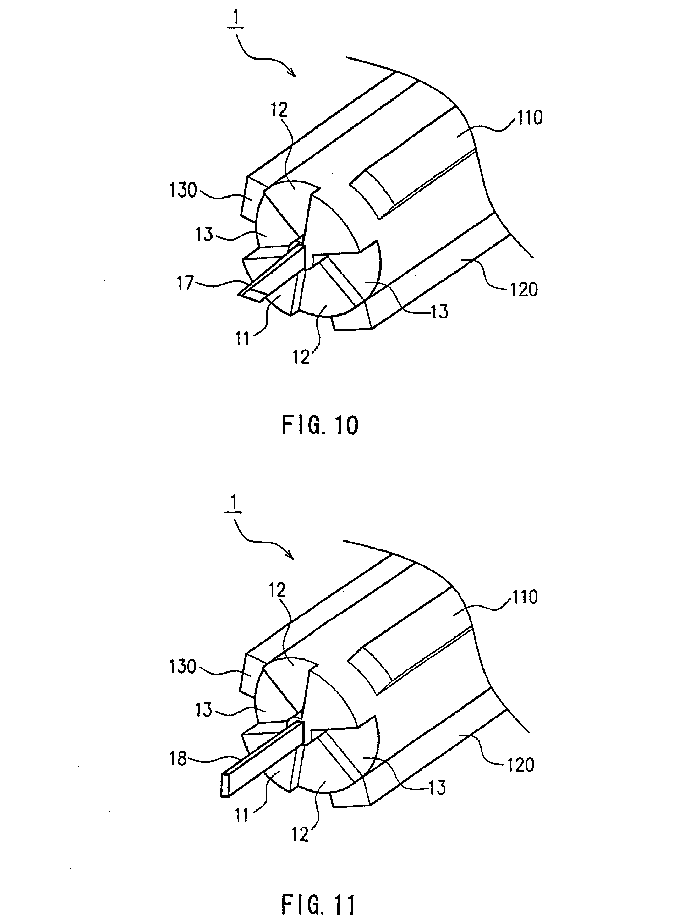

In the example of FIG. 10, a chisel 17 as the protrusible tool is attached to the movable member 1. The chisel 17 shown in FIG. 10 is a flat chisel that is useful for flattening a bottom face of a hole already provided, or for cutting a blind hole with a certain depth. It should be noted that a chisel other than the flat chisel can be used.

A processing operation employing the tool-protruding device shown in FIG. 10 is performed, for instance, in the following manner. In the example shown in FIG. 10 also, as in the example of FIG. 8, the movable member 1 is set in the moving device, the cap is put on the moving device, and the positions of the projections are adjusted by turning the cap so that the length of a portion of the chisel 17 protruded out of the cap is determined.

Next, the user holds the tool-protruding device having the chisel 17 protruded out of the cap, inserts the chisel 17 into a hole to be subjected to the cutting, and carries out the cutting. Here also, as in the example of FIG. 8, the length of a portion of the chisel 17 protruded out of the cap is predetermined, and the cap is brought into contact with surroundings of the hole to be processed, thereby functioning as a stopper. Therefore, the user never excessively cuts the bottom face of the hole. It should be noted that the example shown in FIG. 10 is effective in the case where the hole subjected to the cutting has a complex structure in which a cutting point is present at a complex position, and the dimensional accuracy of the hole is strict, or in the case where another cutting point is present also behind the foregoing cutting point.

In the example shown in FIG. 11, a file 18 as the protrusible tool is attached to the movable member 1. The file 18 is useful for processing or expanding a hole already provided, and strictly adjusting a depth of a hole. Further, in the example shown in FIG. 11, the file 18 is a bar-like file having a rectangular cross section, but it may be a file having a cross section in a shape other than the foregoing, for instance, a circular cross section.

The processing employing the tool-protruding device shown in FIG. 11 is performed, for instance, in the following manner. In the example shown in FIG. 11 also, as in the example of FIG. 8, the movable member 1 is set in the moving device, the cap is put on the moving device, and the positions of the projections are adjusted by turning the cap so that the length of a portion of the file 18 protruded out of the cap is determined.

Next, the user holds the tool-protruding device having the file 18 protruded out of the cap, inserts the file 18 into a hole to be subjected to the cutting, and carries out the cutting. Here also, as in the example of FIG. 8, the length of a portion of the file 18 protruded out of the cap is predetermined, and the cap is brought into contact with surroundings of the hole to be processed, thereby functioning as a stopper. Therefore, the user is required to pay attention only to the expansion of the hole in diameter, without concern for the hole in the depth direction.

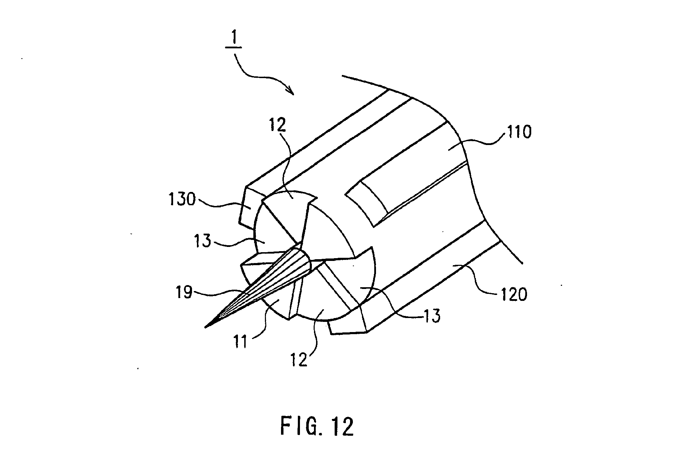

In the example shown in FIG. 12, a reamer19 as the protrusible tool is attached to the movable member 1. It should be noted that the reamer 19 is useful for strictly adjusting the diameter and depth of a hole already provided. Further, the reamer 19 is a taper reamer, but it may be a reamer without taper.

The processing employing the tool-protruding device shown in FIG. 12 is performed, for instance, in the following manner. In the example shown in FIG. 12 also, as in the example of FIG. 8, the movable member 1 is set in the moving device, the cap is put on the moving device, and the positions of the projections are adjusted by turning the cap so that the length of a portion of the reamer 19 protruded out of the cap is determined.

Next, the user holds the tool-protruding device having the reamer 19 protruded out of the cap, inserts the reamer 19 into a hole to be subjected to the cutting, and carries out the work. Here also, as in the example of FIG. 8, the length of a portion of the reamer 19 protruded out of the cap is predetermined, and the cap is brought into contact with surroundings of the hole to be processed, thereby functioning as a stopper. Therefore, the user is required to pay attention only to the expansion of the hole in diameter, without concern for the hole in the depth direction.

As shown in FIGS. 1, 2, and 4 to 12, in the present invention, a needle, a drill, a saw, a chisel, a file, and a reamer are attachable as a protrusible tool to the movable member, but the present invention is not limited to these. Examples of the other protrusible tools include a sharpening stone, a plane, a scraper, a wrench, a cutter, and the like.

### INDUSTRIAL APPLICABILITY

As described above, using the tool-protruding device of the present invention, the user is allowed to easily adjust the protrusion length of the protrusible tool such as a medical instrument or a tool, without carrying out a complex work. Further, since the tool-protruding device of the present invention has a simple structure, it can be manufactured efficiently.

## Claims

1. A tool-protruding device comprising at least a movable member, a moving device for moving the movable member, and a cap attached to the moving device and covering the movable member,
wherein
the movable member has an end that has a shoulder part having a varied height and that is configured so that a protrusible tool is attachable thereto, and
the cap has a through hole that allows the protrusible tool to pass threrethrough, and one or a plurality of projections that project toward center of the through hole, so that the shoulder part of the movable member is brought into contact with the projections, so as to regulate a length of a portion of the protrusible tool protruded out of the cap.

2. The tool-protruding device according to claim 1,
wherein the height of the shoulder part of the movable member is varied stepwise.

3. The tool-protruding device according to claim 2,
wherein
the movable member has a plurality of projected portions that linearly extend in a direction in which the movable member is moved and that correspond to levels of the height of the shoulder part that is varied stepwise, respectively,
the moving device has a groove into which one of the projected portions is to be fitted, and
the shoulder part, at a position where it has a predetermined level of the height that corresponds to one of the projected portions fitted in the groove, is brought into contact with the projections formed in the cap by causing the moving device to move the movable member in a state in which the projected portion is fitted in the groove.

4. The tool-protruding device according to claim 3,
wherein
the movable member has grooves in place of the projected portions, the moving device has a projected portion in place of the groove, and
the projected portion is fitted in the groove.

5. The tool-protruding device according to claim 1,
wherein the height of the shoulder part is varied stepwise at two to twelve levels.

6. The tool-protruding device according to claim 1,
wherein the protrusible tool is a chisel, a sharpening stone, a plane, a scraper, a drill, a saw, a wrench, a cutter, a file, or a reamer.

7. The tool-protruding device according to claim 1,
wherein the moving device ejects and moves the movable member.

8. The tool-protruding device according to claim 1,
wherein the movable member is a cylindrical body composing a lancet.

9. The tool-protruding device according to claim 8,
wherein
the moving device is a device body composing a lancet-ejecting device, and
the cap is a cap composing the lancet-ejecting device.

10. The tool-protruding device according to claim 8,
wherein the protrusible tool is a needle or a cutting edge for the lancet.

11. The tool-protruding device according to claim 8,
wherein a length of a portion of the protrusible tool protruded out of the cap is adjustable in a range of 0.0 mm to 4.0 mm.

12. The tool-protruding device according to claim 1,
wherein
the movable member is a cylindrical body composing a lancet,
the protrusible tool is a needle or a cutting edge for the lancet,
the moving device is a device body composing a lancet-ejecting device, and
the cap is a cap composing the lancet-ejecting device.

13. A lancet comprising:
a cylindrical body; and
a needle or a cutting edge attached to an end of the cylindrical body,
wherein the cylindrical body has a shoulder part at the end, the shoulder part having a varied height.
